# EUROPEAN PATENT SPECIFICATION

(11) **EP 3 785 684 B1**
(45) Date of publication and mention of the grant of the patent: **24.12.2025**
(21) Application number: 20194122.6
(22) Date of filing: 02.09.2020
(51) Int. Cl.: A61F 9/00, A61B 17/02, A61M 5/42

(54) **OCULAR INJECTION ASSIST DEVICE**
VORRICHTUNG ZUR UNTERSTÜTZUNG VON OKULAREN INJEKTIONEN
DISPOSITIF D'ASSISTANCE D'INJECTION OCULAIRE

(30) Priority: 02.09.2019 NL 2023743
(43) Date of publication of application: 03.03.2021
(73) Proprietor: Medical Workshop B.V., 9723 AZ Groningen (NL)
(72) Inventor: VELDHUIZEN, Rudi, 3628 ET Kockengen (NL)
(74) Representative: Nederlandsch Octrooibureau

(56) References cited:
- WO-A1-2008/084063
- WO-A1-2016/083669

## Description

### Field of the invention

The present invention relates to an ocular injection assist device comprising an ocular surface support element having a concave contact surface and a device manipulation element attached to the ocular surface support element.

### Background art

International patent publication WO2007/052730 discloses a fixture for intra-vitreous injection, comprising an injection needle fixture plate with an injection needle insertion hole, and an eyeball fixture for fixing the position of the insertion hole on the eyeball.

International patent publication WO2008/097072 discloses a device for intraocular administration of a substance using a hypodermic needle, comprising a support element which can be placed on the eye. Furthermore directing means are provided for orienting a hypodermic needle relative to the eye, in the form of a bore formed in the support element. The bore has a direction for guiding the needle substantially perpendicular to the eye surface.

International patent publication WO2016/083669 discloses an ocular therapeutics tool having a stabilizer with a hollow body and a base connected to the body. An injection guide is provided which is connectable to the stabilizer and is arranged for receiving an injection needle. A stopper is provided in the injection guide to define the injection depth of a needle inserted into the injection guide.

US patent publication US 9,999,541 discloses a positioning device for use in an examination, procedure or surgery of the eye. The structure includes a curved lateral portion, upper peripheral edge and lower peripheral edge with four indentations. The upper peripheral edge defines an opening having a diameter about commensurate with the cornea, so that when placed on the eye, the inner circumference of upper peripheral edge coincides approximately with the cornea limbus. The positioning device can also include a handle pivotably attached to the incurvate body. The positioning device can be used to stabilize the eye as well as assist in accurate positioning of instruments during eye examination, procedure or surgery involving the anterior or posterior segment of the eye.

US patent publication US2019/046177 discloses an eyelid speculum used to hold the eyelids of a patient's eye open during a medical procedure includes two retractors which are configured to engage the edge of a patient's eyelid and a central opening between the retractors which permits the medical practitioner to view the cornea of a patient's eye during a medical procedure. The eyelid speculum can be provided in a sterile medical kit.

### Summary of the invention

Until recently, intravitreal or intraocular injections by means of a hypodermic needle, by which a substance such as a medication, is injected into a human or animal eye, were only used in exceptional cases. Lately, after the discovery of a new generation of medicines, it has become possible in ophthalmology to use intravitreal injections for treating certain eye disorders, which until now could hardly be therapeutically treated, or only to a limited extent. The eye disorders that can thus be treated are: macular degeneration, vena occlusions, diabetes retinopathy, all kinds of macula oedema, neovascular glaucoma, some forms of ischemic eye disorders, etc. A hypodermic needle that is inserted at an incorrect position or at an incorrect angle may cause complications, such as intraocular haemorrhage, or needle damage to the eye lens, which may in turn cause cataracts, retinal detachment and the like. The present invention seeks to provide a tool that makes it possible, using one instrument, to keep the eye stationary and at the same time makes it easier to give an injection into the eye.

According to claim 1, an ocular injection assist device is provided as defined, further comprising one or more guide indentations are provided on an outer edge of the ocular surface support element, and wherein the device manipulation element comprises a support element for a syringe body.

The present invention seeks to provide a reliable solution wherein the procedure of injecting a particular medication into a human or animal eye can be carried out under identical, reproducible circumstances at all times. This also prevents any undesirable incorrect insertion of the hypodermic needle into the eye and the associated inconveniences to the patient. Thus, the present invention embodiments make it easier for a medical practitioner, for example an ophthalmologist or a surgeon, to carry out eye treatments in an identical, reproducible manner, thereby preventing all kinds of traumas to the eye.

Further embodiments are described by the dependant claims, and with reference to the exemplary embodiments as shown in the drawings.

### Short description of drawings

The present invention will be discussed in more detail below, with reference to the attached drawings, in which:
Fig. 1 shows a perspective view of an ocular injection assist device according to an embodiment of the present invention,
Fig. 2 shows a side perspective view of an ocular injection assist device with a syringe according to a further embodiment of the present invention,
Fig. 3 shows a bottom view of the ocular injection assist device of Fig. 2,
Fig. 4 shows a top view of a further embodiment of the ocular injection assist device,
Fig. 5 A-C show partial detailed top views of a part of an ocular injection assist device according to various exemplary embodiments of the present invention,
Fig. 6 shows a side perspective view of an ocular injection assist device with a syringe according to a further embodiment of the present invention, and
Fig. 7 shows a side perspective view of an ocular injection assist device with a syringe according to an even further embodiment of the present invention.

### Description of embodiments

Some eye diagnostics and treatment of eye diseases require intravitreal or intraocular injections of fluids (dye agent or medication). A treatment example can be intravitreal injection of a medication into the corpus vitreum of the human eye. In such cases, the medication must be administered by an ophthalmologist who has experience in giving intravitreal or intraocular injections. The position where the hypodermic needle is to be inserted into the eye is usually determined on the basis of the ophthalmologist's visual assessment and experience. The present invention relates to a device for aiding in intraocular administration of a substance, for example a medication, into a human or animal eye by means of a hypodermic needle. The device can be simply held by hand of a medical practitioner and be manipulated on the eye for inserting a needle by means of the syringe, e.g. for the purpose of administering a substance or medicine.

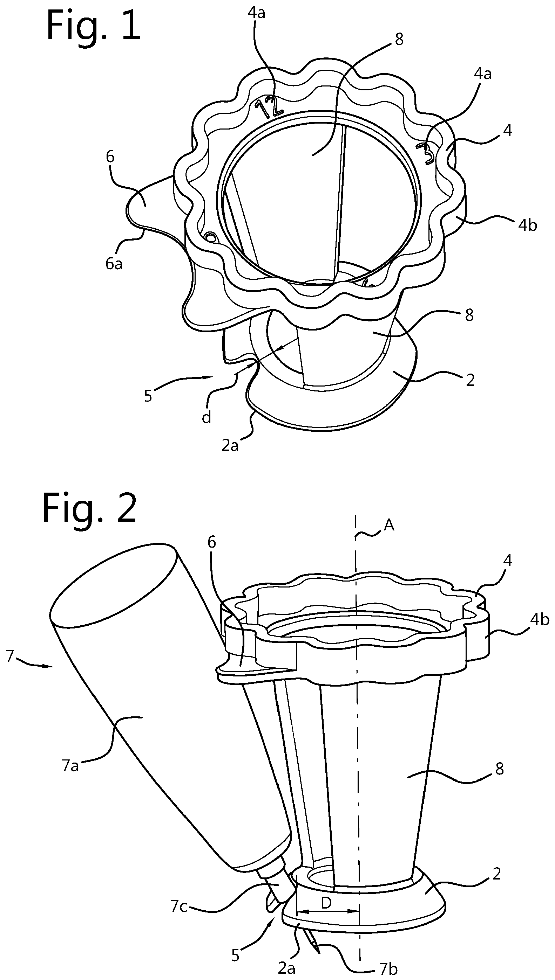

The present invention embodiments relate to an ocular injection assist device 1 comprising an ocular surface support element 2 having a concave contact surface 3 and a device manipulation element 4 attached to the ocular surface support element 2. Fig. 1 shows a schematic representation of the ocular injection assist device 1 according to an embodiment of the present invention. The ocular injection assist device 1 comprises an ocular surface support element 2 located at a proximal end and a device manipulation element 4 at a proximal end of the ocular injection assist device 1. As shown in the embodiment of Fig. 1, the ocular surface support element 2 has an annular shape with an aperture in the middle, allowing unobstructed view on/through the eye lens during use. Also, the annular shape ensures the contact area with the eye is minimal, ensuring stable contact with the convex eye surface without irritating the eye unnecessarily. Further, the detailed features of the ocular surface support element 2 allow to have an improved and reproducible orientation of a needle when the ocular injection assist device 1 is used in conjunction with e.g. a syringe. The concave contact surface 3 of the ocular surface support element 2 is congruent with a regular generally spherical/convex eye surface. In other words, the concave contact surface 3 is designed to have a radius that is substantially similar to the radius of a human or an animal eye. The ocular surface support element 2 is placed on the eye and is also useable for keeping the eye open during treatment. The ocular surface support element 2 thus enables a stable placement and orientation of the device 1 on the convex eye surface with a minimal area of contact, so that irritation to the patient's eye is reduced as much as possible. The ocular surface support element 2 also helps the patient from blinking the eye involuntarily and thus disturb the treatment area for the ophthalmologist, resulting in incorrect insertion of the hypodermic needle into the eye.

To make it possible to carry out repeated medical treatments into the eye, one or more guide indentations 5 are provided on an outer edge 2a of the ocular surface support element 2. The one or more guide indentations 5 helps to guide a secondary device, such as a syringe needle 7b (see e.g. the perspective view of Fig. 2). The shape of the one or more guide indentations 5 can be different, but all have an outward edge with an apex at an outward distance d from an inner edge of the ocular surface support element 2, allowing to precisely position a needle 7b on the ocular surface. Normally, reference is made to the limbus of the eye, which during use can be aligned with the inner edge of the ocular surface support element 2.

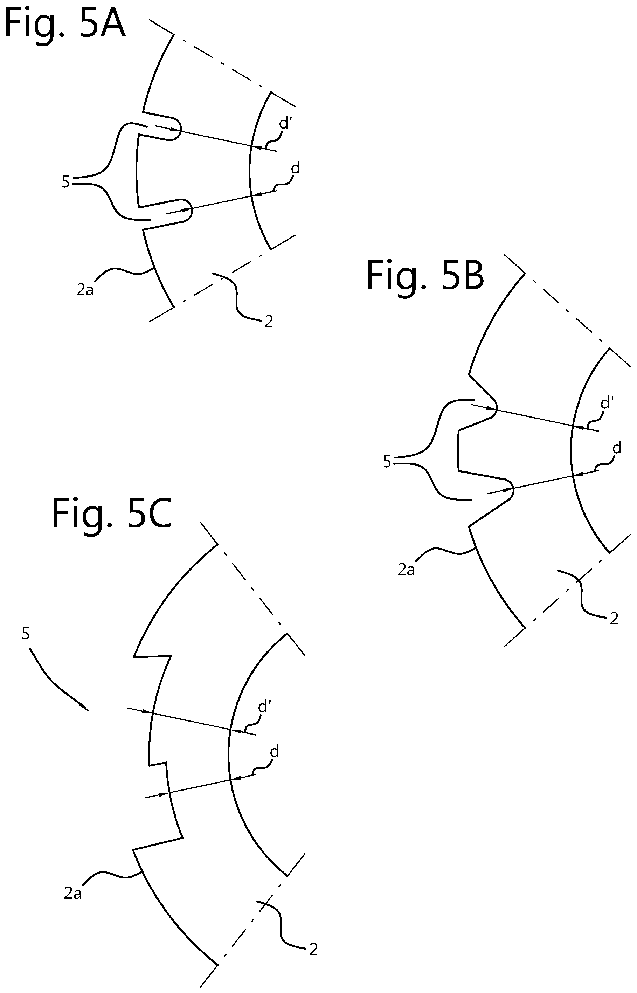

In a further embodiment of the present invention, the one or more guide indentations 5 comprise a profiled edge 2a of the ocular surface support element 2. In the partial detailed top views of Fig. 5 A-C, various embodiments of an ocular injection assist device 1 are shown, in particular parts of the ocular surface support element 2 having different shapes as for the profiled edge 2a. In an embodiment, the profiled edge 5a comprises one or more of a wedge shape, a slit shape, a recessed circumference shape. The embodiment of Fig. 5A shows a slit shape, Fig. 5B shows a wedge shape and Fig. 5C shows a recessed circumference shape.

In further embodiments, an apex of the profiled edge 5a is provided at a predetermined distance d, d' from an inner edge of the ocular support element 2. In other words, the one or more guide indentations 5 can be provided with their respective apex at a predetermined distance d, d' from an (inner) edge of the ocular surface support element 2. The distances d and d' are measured as distances between the inner edge of the ocular surface support element 2 and the apex of the respective guide indentation 5, and are e.g. equal to 3.5 and 4 mm. These distances correspond to prescribed distances for the injection needle measured from the limbus (the change from iris of the eye to the (white) eye surface). The distance of 3.5mm is prescribed for patients with a regular eye, and 4 mm for patients having an intra ocular lens. By aligning the inner edge of the ocular support element 2 with the limbus, the correct injection site can be easily determined using this structure of the ocular injection assist device.

The above described embodiments allow to accommodate needles of different diameters and/or shapes, and various pre-set injection positions into a single ocular injection assist device 1.

The one or more guide indentations 5 have an apex radius of less than 3 mm, e.g. less than 1 mm in further exemplary embodiments of the present invention. The selection of the apex radius can depend on parameters such as the diameter of the needle 7b (e.g. a 22 Gauge (0.72mm diameter) needle, or a 30 Gauge (0.31mm diameter) needle) or the diameter of the needle attachment part 7c of the syringe 7 that is used in combination with the ocular injection assist device 1. In embodiments where the ocular injection assist device has more than one guide indentation 5, the apex radius of each of the guide indentations 5 can be different. In an exemplary embodiment of the ocular injection assist device 1, two guide indentations 5 are provided on two opposite sides of the ocular surface support element 2.The device manipulation element 4 allows the health practitioner to manipulate or fine tune the position of the ocular injection assist device 1 before injecting the medication into the eye. In an exemplary embodiment of the present invention, the device manipulation element 4 comprises a substantially ring shaped element positioned remote from the ocular surface support element 2. In the exemplary embodiment shown in Fig. 1, the ocular surface support element 2 has a smaller outer diameter than the device manipulation element 4. The ring shaped element of the device manipulation element 4 is open and it allows the health practitioner to visually inspect the eye during use of the ocular injection assist device 1. The ocular surface support element 2 and the device manipulation element 4 in this exemplary embodiment are mechanically connected to each other by a strut element 8. In a further embodiment of the present invention, the device manipulation element 4 is attached to the ocular surface support element 2 by one or more strut elements 8. In the embodiment shown in Fig. 1, two strut elements 8 are placed on opposite sides, connecting the ocular surface support element 2 and the device manipulation element 4 in a reliable and structural manner. The openings between the two strut elements 8 also allow the health practitioner to e.g. insert additional tools such as a knife, if necessary, during the treatment.

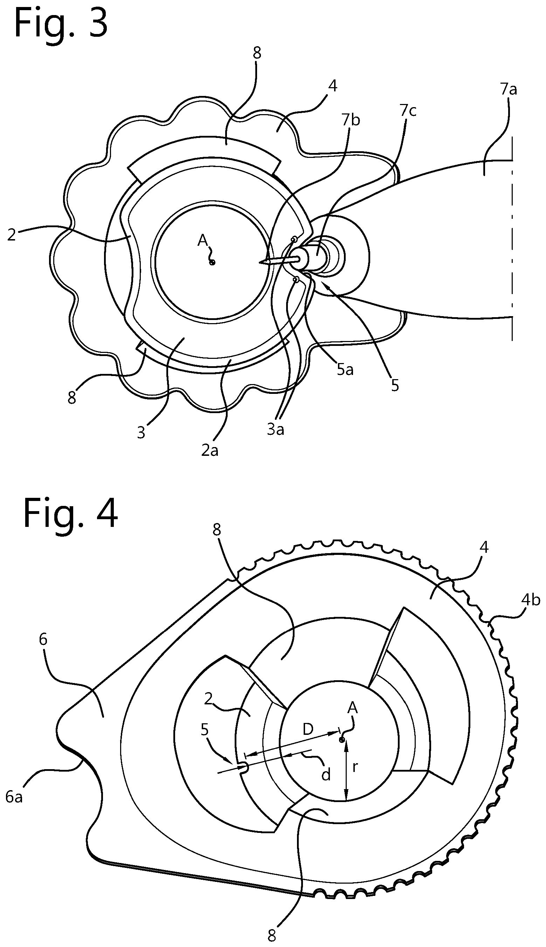

In an embodiment of the present invention, the one or more guide indentations 5 are each provided at a respective predetermined distance D from a longitudinal axis A of the ocular injection assist device 1, the longitudinal axis A extending through a center point of the ocular surface support element 2 and a center point of the device manipulation element 4. Note that the center points are virtual center points, as both the ocular surface support element 2 and device manipulation element 4 have an opening to allow view on the eye during use. Furthermore, it is noted that during use, the ocular injection assist device 1 need not be centered on the eyeball. The predetermined distance 'D' is defined relative to the longitudinal axis A, and is equal to the sum of a radius 'r' of the opening in the ocular surface support element 2 and an outward distance 'd' from the inner edge of the ocular surface support element 2 to the apex of the guide indentation 5. This is best visible in the bottom view of the exemplary embodiment shown in Fig. 4.

Fig. 2 shows a side perspective view of the ocular injection assist device 1 of Fig. 1 in use with a syringe 7 having a syringe needle 7b positioned in the guide indentation 5. Usually, type 20 Gauge - 30 Gauge hypodermic needles are used for intravitreal or intraocular injections. The needle 7b is usually inserted into specific locations of the eye such as at the pars plana, at a distance of a few millimetres, e.g. 4 mm, from the limbus of the eye. As described above, the apex of the one or more indentations 5 as at an outward distance d from the inner edge of the ocular surface support element 2. In exemplary embodiments, the outward distance d is set at 3.5 or 4 mm, allowing the needle 7b to be inserted at a distance of 3.5 or 4 mm, respectively from the limbus of the eye, if the inner edge of the ocular surface support element 2 is aligned with the limbus. The one or more guide indentations 5 can be adapted to accommodate needles 7b (or similar injection components) of different diameters or for inserting needles of different depths. This makes it possible to use the ocular injection assist device 1 for various syringes and hypodermic needles in dependence on the medical treatment to be carried out.

The one or more guide indentations 5 can be designed to guide either the tip end of a syringe needle 7b or a needle attachment part 7c of a syringe 7 (see exemplary embodiment of Fig. 2).

As shown in the side perspective view of Fig. 2, the ocular surface support element 2 has a guide indentation 5 on one of its side wherein a needle 7b attached to a syringe 7 can be guided. As shown in Fig. 2 (bit also visible in the perspective view of Fig. 1), in this exemplary embodiment of the present invention ocular injection assist device 1, the device manipulation element 4 comprises a support element 6 for a syringe body 7a, in the form of a support arm 6. This allows the syringe body 7a to rest on the device manipulation element 4, restricting it from having any lateral movement. According to a further embodiment of the present invention, the support arm 6 has an outward directed profile 6a congruent to an outer surface of the syringe body 7a. The outward directed profile 6a can have different shapes, e.g. circular, oval or elliptical. The exemplary embodiment in Fig. 2 shows an outward directed profile 6a having a circular arc shape. The outward directed profile 6a helps for a precise alignment of the angle of the needle 7b with respect to the ocular surface of the eye. The relative positioning of support arm 6 (or outward directed profile 6a) and (the apex) of the one or more guide indentations 5 is selected in such a manner that the needle 7b automatically takes up a substantially perpendicular orientation relative to the eye surface during use. The angle between the needle 7b and the eye surface may range between 70° and 95°. In this way a reproducible and accurate placement of the needle 7b on the eye surface is ensured at all times. This prevents trauma to the eye of the patient, whilst preventing the risk of complications resulting from incorrect insertion or insertion at an incorrect angle of the hypodermic needle 7b, such as intraocular haemorrhage, needle damage to the eye lens, retinal detachment, etc. As the predetermined distance D (between apex of indentation 5 and longitudinal axis A) is set by the desired injection position (3.5 or 4 mm from the limbus), the actual angle of the syringe 7 (or needle 7b) is determined by the radius of the syringe body 7a in combination with the positioning of the outward directed profile 6a relative to the longitudinal axis A. In a further embodiment of the present invention, the support arm 6 is extending to a predetermined distance profile from the device manipulation element 4. The predetermined distance can be as measured perpendicular to the longitudinal axis A. The dimensions of the support arm 6 in combination with the relative positioning of the one or more guide indentations 5 ensure a proper orientation of the syringe 7 or needle 7b on the ocular surface of the eye, e.g. perpendicular to the concave contact surface 3 of the ocular surface support element 2.

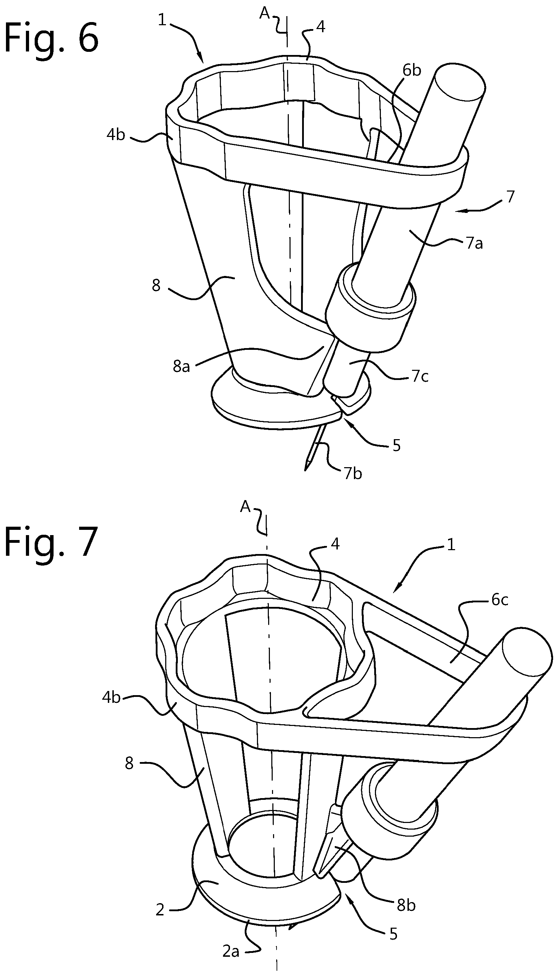

In further embodiments, shown in the perspective views in Fig. 6 and 7, alternative features are present which enhance the support characteristics of the ocular injection assist device 1. As opposed to the embodiment shown and discussed with reference to Fig. 2 above, the support element 6 in these embodiments has an inward directed profile 6b; 6c congruent to an outer surface of the syringe body 7a. Furthermore, an additional element is present to support the syringe 7 closer to the guide indentations 5, i.e. the one or more strut elements 8 (between device manipulation element 4 and ocular surface support ring 2) further comprise an outwardly directed syringe head support element 8a; 8b, i.e. directed outwardly from the longitudinal axis A.

In the embodiment shown in Fig. 6, the device manipulation element 4 comprises a partial substantially ring shaped element in combination with the support element 6, both positioned remote from the ocular surface support element 2. As shown, the device manipulation element 4 and support element 6 together form a band type, stretched and rounded part at the top of the ocular injection assist device 1, wherein the support element 6 comprises an inward directed profile 6b congruent with an outside surface of syringe body 7a. This allows to first position the ocular injection assist device 1 on the eye of a patient, and then easily move the syringe 7 into position from the open top of the device 1. In addition, the one or more struts 8 are locally stretched outward to provide the outwardly directed syringe head support element 8a. A further advantage of the embodiment shown in Fig. 6 is that it can still be manufactured easily with e.g. injection moulding techniques.

In the embodiment shown in Fig. 7, the device manipulation element 4 is (similar to the embodiment shown in Fig. 2) a generally circular element at the top of the ocular injection assist device 1, onto which the support element 6 is attached. The support element 6 is implemented as a bent band type element, provided with an inward directed profile 6c, part of which is congruent with an outside surface of syringe body 7a. This configuration still leaves sufficient open space to allow the user to bring the syringe 7 into position properly. The support element 6 can be attached (connected, glued, etc.) onto the device manipulation element 4, but is can also be provided as an integrated part. e.g. using (injection) moulding techniques. In this embodiment, furthermore, the one or more strut elements 8 (between device manipulation element 4 and ocular surface support ring 2) is further provided with an outwardly directed syringe head support element 8b, extending from the respective strut element 8.

Note that the various element alternatives as discussed above in general with reference to the embodiments shown in Fig. 2, 6 and 7, may be interchanged to provide even further alternative embodiments.

A further embodiment of the present invention relates to an ocular injection assist device, wherein the device manipulation element 4 comprises one or more orientation markers 4a. The orientation markers 4a can be visual indications that aids the health practitioner for a proper orientation of the ocular injection assist device 1 with respect to the ocular surface of the eye. The orientation markers 4a can be any type of indication such as numbers, symbols, structures etc. Fig. 1 shows exemplary orientation markers 4a as numbers 3, 6, 9, 12 placed as a clock indices on an upper surface of the ring shaped element embodiment of the device manipulation element 4.

A further embodiment of the present invention relates to an ocular injection assist device, wherein the concave contact surface 3 comprises a plurality of extending elements 3a. Fig. 3 shows a bottom view of the exemplary embodiment of Fig. 2, wherein these features are visible. The plurality of extending elements 3a are e.g. implemented as two or three extending elements 3a near to the guide indentations 5 provided on the concave contact surface 3. The extending elements 3a function to keep the ocular injection assist device 1 at the correct position on the external surface of an eye during use. Using this exemplary embodiment, a hypodermic needle 7b can be inserted into the eye in a reliable and reproducible manner.

Fig. 4 shows a top view of a further embodiment of the ocular injection assist device 1. Herein, the ocular injection assist device is provided wherein the device manipulation element 4 comprises a textured outer surface 4b. The textured outer surface 4b can be implemented as a grooved surface, as shown in the embodiment of Fig. 4, or alternatively can be provided as a corrugated outer surface such as shown in the embodiments of Fig. 1-3.The textured outer surface 4b provides a good grip to hold the device manipulation element 4, thereby preventing the ocular injection assist device 1 from slipping away from the hand of the health practitioner. The textured outer surface 4b can be designed in various alternative or additional profiles such as a saw tooth profile, or a flat roughened surface.

The present invention has been described above with reference to a number of exemplary embodiments as shown in the drawings. Modifications and alternative implementations of some parts or elements are possible, and are included in the scope of protection as defined in the appended claims.

## Claims

1. An ocular injection assist device (1) comprising
an ocular surface support element (2) having a concave contact surface (3), and
a device manipulation element (4) attached to the ocular surface support element (2),
wherein one or more guide indentations (5) are provided on an outer edge (2a) of the ocular surface support element (2) for guiding a needle (7b) attached to a syringe body (7a), **characterized in that**
the device manipulation element (4) comprises a support element (6) for the syringe body (7a),
wherein the support element (6) is extending away from the device manipulation element (4), and is arranged to receive the syringe body (7a).

2. The ocular injection assist device according to claim 1, wherein the one or more guide indentations (5) are each provided at a respective predetermined distance (D) from a longitudinal axis (A) of the ocular injection assist device (1), the longitudinal axis (A) extending through a center point of the ocular surface support element (2) and a center point of the device manipulation element (4).

3. The ocular injection assist device according to claim 1 or 2, wherein the support element (6) has an outward directed profile (6a) congruent to an outer surface of the syringe body (7a).

4. The ocular injection assist device according to claim 1 or 2, wherein the support element (6) has an inward directed profile (6b; 6c) congruent to an outer surface of the syringe body (7a).

5. The ocular injection assist device according to any one of claims 1-4, wherein the support element (6) is extending to a predetermined distance profile from the device manipulation element (4).

6. The ocular injection assist device according to any one of claims 1-5, wherein the device manipulation element (4) comprises a substantially ring-shaped open element.

7. The ocular injection assist device according to any one of claims 1-6, wherein the device manipulation element (4) is attached to and positioned remote from the ocular surface support element (2) by one or more strut elements (8).

8. The ocular injection assist device according to any one of claim 1-7, wherein the one or more strut elements (8) further comprise an outwardly directed syringe head support element (8a; 8b)

9. The ocular injection assist device according to any one of claims 1-8, wherein the one or more guide indentations (5) comprise a profiled edge (5a) of the ocular surface support element (2).

10. The ocular injection assist device according to any one of claims 1-9, wherein the profiled edge (5a) comprises one or more of a wedge shape, a slit shape, a recessed circumference shape.

11. The ocular injection assist device according to claim 9 or 10, wherein an apex of the profiled edge (5a) is provided at a predetermined distance (d, d') from an inner edge of the ocular surface support element (2).

12. The ocular injection assist device according to any one of claims 1-11, wherein the one or more guide indentations (5) have an apex radius of less than 3 mm, e.g. less than 1 mm.

13. The ocular injection assist device according to any one of claims 1-12, wherein the device manipulation element (4) comprises one or more orientation markers (4a).

14. The ocular injection assist device according to any one of claims 1-13, wherein the concave contact surface (3) comprises a plurality of extending elements (3a).

15. The ocular injection assist device according to any one of claims 1-14, wherein the device manipulation element (4) comprises a textured outer surface (4b).

## Patentansprüche

1. Gerät zur Unterstützung bei Augeninjektionen (1), das Folgendes aufweist
ein Augenoberflächen-Stützelement (2), das eine konkave Kontaktoberfläche (3) hat, und
ein Gerätemanipulationselement (4), das an dem Augenoberflächen-Stützelement (2) angebracht ist, wobei ein oder mehrere Führungsvertiefungen (5) an einem äußeren Rand (2a) des Augenoberflächen-Stützelements (2) für das Führen einer Nadel (7b), die an einem Spritzenkörper (7a) angebracht ist, bereitgestellt sind, **dadurch gekennzeichnet, dass**
das Gerätemanipulationselement (4) ein Stützelement (6) für den Spritzenkörper (7a) umfasst, wobei das Stützelement (6) sich weg vom Gerätemanipulationselement (4) erstreckt, und angeordnet ist, um den Spritzenkörper (7a) aufzunehmen.

2. Gerät zur Unterstützung bei Augeninjektionen nach Anspruch 1, wobei die eine oder die mehreren Führungsvertiefungen (5) jede in einem jeweiligen vorbestimmten Abstand (D) von einer Längsachse (A) des Geräts zur Unterstützung bei Augeninjektionen (1) bereitgestellt sind, wobei sich die Längsachse (A) durch einen Mittelpunkt des Augenoberflächen-Stützelements (2) und einen Mittelpunkt des Gerätemanipulationselements (4) erstreckt.

3. Gerät zur Unterstützung bei Augeninjektionen nach Anspruch 1 oder 2, wobei das Stützelement (6) ein nach außen gerichtetes Profil (6a) hat, das deckungsgleich mit einer äußeren Oberfläche des Spritzenkörpers (7a) ist.

4. Gerät zur Unterstützung bei Augeninjektionen nach Anspruch 1 oder 2, wobei das Stützelement (6) ein nach innen gerichtetes Profil (6b; 6c) hat, das deckungsgleich mit einer äußeren Oberfläche des Spritzenkörpers (7a) ist.

5. Gerät zur Unterstützung bei Augeninjektionen nach einem der Ansprüche 1 bis 4, wobei das Stützelement (6) sich zu einem vorbestimmten Abstandsprofil von dem Gerätemanipulationselement (4) erstreckt.

6. Gerät zur Unterstützung bei Augeninjektionen nach einem der Ansprüche 1 bis 5, wobei das Gerätemanipulationselement (4) ein im Wesentlichen ringförmiges offenes Element umfasst.

7. Gerät zur Unterstützung bei Augeninjektionen nach einem der Ansprüche 1 bis 6, wobei das Gerätemanipulationselement (4) angebracht ist am und entfernt angeordnet ist von dem Augenoberflächen-Stützelement (2) durch ein oder mehrere Strebenelemente (8).

8. Gerät zur Unterstützung bei Augeninjektionen nach einem der Ansprüche 1 bis 7, wobei ferner das eine oder die mehreren Strebenelemente (8) ein nach außen gerichtetes Spritzenkopf-Stützelement (8a; 8b) umfassen.

9. Gerät zur Unterstützung bei Augeninjektionen nach einem der Ansprüche 1 bis 8, wobei die eine oder die mehreren Führungsvertiefungen (5) einen profilierten Rand (5a) des Augenoberflächen-Stützelements (2) umfassen.

10. Gerät zur Unterstützung bei Augeninjektionen nach einem der Ansprüche 1 bis 9, wobei der profilierte Rand (5a) eine oder mehrere von einer Keilform, einer Schlitzform, einer umlaufend vertieften Kreisform umfasst.

11. Gerät zur Unterstützung bei Augeninjektionen nach Anspruch 9 oder 10, wobei ein Scheitelpunkt des profilierten Randes (5a) in einem vorbestimmten Abstand (d, d') von einem inneren Rand des Augenoberflächen-Stützelements (2) bereitgestellt ist.

12. Gerät zur Unterstützung bei Augeninjektionen nach einem der Ansprüche 1 bis 11, wobei die eine oder die mehreren Führungsvertiefungen (5) einen Scheitelpunktradius von kleiner als 3 mm, z.B. kleiner als 1 mm, haben.

13. Gerät zur Unterstützung bei Augeninjektionen nach einem der Ansprüche 1 bis 12, wobei das Gerätemanipulationselement (4) eine oder mehrere Orientierungsmarkierungen (4a) umfasst.

14. Gerät zur Unterstützung bei Augeninjektionen nach einem der Ansprüche 1 bis 13, wobei die konkave Kontaktoberfläche (3) eine Vielzahl von Erstreckungselementen (3a) umfasst.

15. Gerät zur Unterstützung bei Augeninjektionen nach einem der Ansprüche 1 bis 14, wobei das Gerätemanipulationselement (4) eine strukturierte äußere Oberfläche (4b) umfasst.

## Revendications

1. Dispositif d'assistance d'injection oculaire (1) comprenant
un élément de support de surface oculaire (2) ayant une surface de contact concave (3), et
un élément de manipulation de dispositif (4) fixé à l'élément de support de surface oculaire (2),
où une ou plusieurs encoches de guidage (5) sont prévues sur un bord extérieur (2a) de l'élément de support de surface oculaire (2) pour guider une aiguille (7b) fixée à un corps de seringue (7a), **caractérisé en ce que**
l'élément de manipulation de dispositif (4) comprend un élément de support (6) pour le corps de seringue (7a),
où l'élément de support (6) s'étend de façon à s'écarter de l'élément de manipulation de dispositif (4) et est agencé pour recevoir le corps de seringue (7a).

2. Dispositif d'assistance d'injection oculaire selon la revendication 1, où l'une ou plusieurs encoches de guidage (5) sont chacune prévues à une distance prédéterminée respective (D) d'un axe longitudinal (A) du dispositif d'assistance d'injection oculaire (1), l'axe longitudinal (A) s'étendant à travers un point central de l'élément de support de surface oculaire (2) et un point central de l'élément de manipulation de dispositif (4).

3. Dispositif d'assistance d'injection oculaire selon la revendication 1 ou 2, où l'élément de support (6) présente un profil dirigé vers l'extérieur (6a) congruent à une surface extérieure du corps de seringue (7a).

4. Dispositif d'assistance d'injection oculaire selon la revendication 1 ou 2, où l'élément de support (6) présente un profil dirigé vers l'intérieur (6b ; 6c) congruent à une surface extérieure du corps de seringue (7a).

5. Dispositif d'assistance d'injection oculaire selon l'une quelconque des revendications 1 à 4, où l'élément de support (6) s'étend jusqu'à un profil de distance prédéterminé à partir de l'élément de manipulation de dispositif (4).

6. Dispositif d'assistance d'injection oculaire selon l'une quelconque des revendications 1 à 5, où l'élément de manipulation de dispositif (4) comprend un élément ouvert sensiblement en forme d'anneau.

7. Dispositif d'assistance d'injection oculaire selon l'une quelconque des revendications 1 à 6, où l'élément de manipulation de dispositif (4) est fixé à l'élément de support de surface oculaire (2) et positionné à distance de celui-ci par un ou plusieurs éléments d'entretoise (8).

8. Dispositif d'assistance d'injection oculaire selon l'une quelconque des revendications 1 à 7, où l'un ou plusieurs éléments d'entretoise (8) comprennent en outre un élément de support de tête de seringue (8a ; 8b) dirigé vers l'extérieur.

9. Dispositif d'assistance d'injection oculaire selon l'une quelconque des revendications 1 à 8, où l'une ou plusieurs encoches de guidage (5) comprennent un bord profilé (5a) de l'élément de support de surface oculaire (2).

10. Dispositif d'assistance d'injection oculaire selon l'une quelconque des revendications 1 à 9, où le bord profilé (5a) comprend une ou plusieurs parmi une forme en coin, une forme en fente, une forme en circonférence en retrait.

11. Dispositif d'assistance d'injection oculaire selon la revendication 9 ou 10, où un sommet du bord profilé (5a) est prévu à une distance prédéterminée (d, d') d'un bord intérieur de l'élément de support de surface oculaire (2).

12. Dispositif d'assistance d'injection oculaire selon l'une quelconque des revendications 1 à 11, où l'une ou plusieurs encoches de guidage (5) ont un rayon de sommet inférieur à 3 mm, par exemple inférieur à 1 mm.

13. Dispositif d'assistance d'injection oculaire selon l'une quelconque des revendications 1 à 12, où l'élément de manipulation de dispositif (4) comprend un ou plusieurs marqueurs d'orientation (4a).

14. Dispositif d'assistance d'injection oculaire selon l'une quelconque des revendications 1 à 13, où la surface de contact concave (3) comprend une pluralité d'éléments d'extension (3a).

15. Dispositif d'assistance d'injection oculaire selon l'une quelconque des revendications 1 à 14, où l'élément de manipulation de dispositif (4) comprend une surface extérieure texturée (4b).
